**Europäisches Patentamt**

**European Patent Office**

**Office européen des brevets**

(19)

(11) Veröffentlichungsnummer: **0 089 565**
B1

(12) # EUROPÄISCHE PATENTSCHRIFT

(45) Veröffentlichungstag der Patentschrift:
**22.05.85**

(21) Anmeldenummer: **83102383.3**

(22) Anmeldetag: **11.03.83**

(51) Int. Cl.⁴: **C 07 C 51/15,** C 07 C 65/05,
C 07 C 65/11

(54) **Verfahren zur Herstellung von aromatischen Hydroxycarbonsäuren.**

(30) Priorität: **23.03.82 DE 3210599**

(43) Veröffentlichungstag der Anmeldung:
**28.09.83 Patentblatt 83/39**

(45) Bekanntmachung des Hinweises auf die Patenterteilung:
**22.05.85 Patentblatt 85/21**

(84) Benannte Vertragsstaaten:
**CH DE FR GB LI**

(56) Entgegenhaltungen:
**DE - A - 1 643 544**
**DE - A - 2 926 694**
**DE - B - 1 493 881**
**DE - C - 960 206**

(73) Patentinhaber: **BAYER AG, Konzernverwaltung RP
Patentabteilung, D-5090 Leverkusen 1 Bayerwerk (DE)**

(72) Erfinder: **Stopp, Gerhard, Dr., Walter-Flex-Strasse 21,
D-5090 Leverkusen (DE)**
Erfinder: **Karkossa, Horst, Immigrather Strasse 57,
D-5653 Leichlingen (DE)**
Erfinder: **Trescher, Viktor, Dr., Voelklinger Strasse 7,
D-5090 Leverkusen (DE)**

## Beschreibung

Die Erfindung betrifft ein Verfahren zur Herstellung von aromatischen Hydroxycarbonsäuren durch Umsetzung in fester Phase von Alkalisalzen aromatischer Hydroxyverbindungen mit Kohlendioxid.

Die Umsetzung von Alkalisalzen aromatischer Hydroxyverbindungen in fester Form mit Hilfe von Kohlendioxid zu den Alkalisalzen der entsprechenden aromatischen Hydroxycarbonsäuren ist bekannt. So wird z.B. in der DE-PS Nr. 960206 ein Verfahren zur Umwandlung von feinkörnigen Alkalisalzen aromatischer Monohydroxyverbindungen in die Alkalisalze der entsprechenden Hydroxycarbonsäuren mit Hilfe von Kohlendioxid beansprucht, das dadurch gekennzeichnet ist, dass die Ausgangssalze in Form von Partikeln mit einem Durchmesser von 20 bis 200 μm eingesetzt und in einem flüssigkeitsähnlichen Zustand, einer sogenannten Wirbelschicht, mit Kohlendioxid begast werden.

Weiterhin ist aus der DE-AS Nr. 1493881 bekannt, p-Hydroxybenzoesäure durch Reaktion von Kaliumphenolat bei einer Temperatur von 190 bis 210° C und einem Druck bis zu 6 atü unter Verwendung eines auf Reaktionstemperatur gebrachten Kreislaufs, bestehend aus Kohlendioxid und Inertgas, herzustellen, wobei laufend für die Abführung der Reaktionswärme und des entstandenen Phenols Sorge getragen und verbrauchtes Kohlendioxid durch Frischgas ersetzt wird und wobei man das Kaliumphenolat in Form von wasserfreiem Granulat oder Presslingen mit bis zu 30 mm Durchmesser oder Kantenlänge einsetzt. Die Reaktion erfolgt dabei in einem Festbettreaktor, der mit dem Kaliumphenolat beschickt ist und bei dem das Gas von unten nach oben geführt wird.

In der GB-PS Nr. 1205447 wird ein Verfahren beschrieben zur Herstellung von Alkalisalzen aromatischer Hydroxycarbonsäuren durch Umsetzung bei höherer Temperatur von Kohlendioxid mit pulverförmigen Alkalimetallphenolaten, die einen Durchmesser von weniger als 70 μm haben.

Aus der DE-OS Nr. 2926694 (US-PS Nr. 4171453) ist ausserdem bekannt, durch Carbonisieren eines trockenen Alkalimetallphenolats in fester Phase mit Kohlendioxid unter Druck das Alkalimetallcarboxylat des entsprechenden Phenols herzustellen. Dieses Verfahren wird so durchgeführt, dass in der ersten Stufe Kohlendioxid mit feinverteiltem, festem Alkalimetallphenolat bei einer Temperatur unter 135° C umgesetzt wird, bis mindestens 25% der stöchiometrischen Menge an Kohlendioxid von dem Phenolat absorbiert sind und in der zweiten Stufe durch Erhöhung der Temperatur auf über 135° C die Carbonisierung des Phenolats weitergeführt wird.

Nachteilig bei den aus dem Stand der Technik bekannten Verfahren sind die z.T. unbefriedigenden Ausbeuten an aromatischen Hydroxycarbonsäuren (DE-OS Nr. 2926694, DE-AS Nr. 1493881 und GB-PS Nr. 1205447), die Probleme, die sich beim Einsatz von pulverförmigem Ausgangsprodukt ergeben, wie der aus Sicherheitsgründen mit einem hohen technischen Aufwand verbundenen Handhabung des pulverförmigen Materials (DE-PS Nr. 960206, GB-PS Nr. 1205447 und DE-OS Nr. 2926694). Weiterhin ist nachteilig, dass unter den in der DE-PS Nr. 960206 beschriebenen Bedingungen durch das Auftreten von flüssigem Phenol die Wirbelschicht durch Verklebung erheblich gestört wird, was auch durch Zugabe von porösen Substanzen, wie Kaolin, nicht ausreichend verhindert werden kann.

Es wurde nun ein Verfahren zur Herstellung von aromatischen Hydroxycarbonsäuren durch Umsetzung in fester Phase von Alkalisalzen aromatischer Hydroxyverbindungen mit Kohlendioxid gefunden, das dadurch gekennzeichnet ist, dass man die Alkalisalze aromatischer Hydroxyverbindungen in Form von Granulat in einem Wirbelbett mit Kohlendioxid bei ggf. erhöhten Drucken zunächst bei Temperaturen von 20 bis 130° C umsetzt, bis sich mindestens 40% der Hydroxyverbindung in das entsprechende Carbonat umgewandelt haben, anschliessend bei Temperaturen von 135 bis 300° C die Reaktion zu Ende führt und danach das erhaltene Reaktionsprodukt in üblicher Weise in die freie Säure überführt.

Als Alkalisalze aromatischer Hydroxyverbindungen können in das erfindungsgemässe Verfahren die Natrium- und/oder Kaliumsalze des Phenols, der Kresole, der Naphthole, des 2-Hydroxycarbazols oder des 3-Hydroxydiphenylenoxids eingesetzt werden. Die aromatischen Hydroxyverbindungen können durch niederes Alkyl, wie Methyl, Ethyl oder tert.-Butyl, sowie durch Halogene, wie Fluor, Chlor, Brom, bevorzugt Chlor, einfach oder mehrfach substituiert sein. Bevorzugt werden in das erfindungsgemässe Verfahren Natriumphenolat, die Natriumsalze der Kresole sowie die Natriumsalze der Chlorphenole, besonders bevorzugt Natriumphenolat, eingesetzt.

Die Alkalisalze der aromatischen Hydroxyverbindungen werden in das erfindungsgemässe Verfahren in Form von wasserfreiem Granulat mit einem Durchmesser von etwa 0,2 bis 5, bevorzugt 0,3 bis 4 mm, und einer inneren Oberfläche von 1 bis 6, bevorzugt 2 bis 4 m²/g Granulat (Oberfläche bestimmt nach BET-Methode/DIN 66 132) eingesetzt.

Das erfindungsgemäss einzusetzende Granulat kann hergestellt werden nach einem eigenen, älteren Verfahren, das in der noch nicht veröffentlichten deutschen Patentanmeldung P Nr. 3206236.2 dargelegt ist.

Nach dem erfindungsgemässen Verfahren setzt man die granulierten Salze der aromatischen Hydroxyverbindungen in einem Wirbelbett mit dem Kohlendioxid, welches ggf. Inertgas, wie Stickstoff, enthalten kann, um. Das Kohlendioxid wird dabei im Kreis gefahren, wobei die Reaktionswärme abgeführt wird. Dem im Kreis geführten Kohlendioxid wird jeweils die Menge an Kohlendioxid wieder zugesetzt, die während der Umsetzung verbraucht worden ist.

Erfindungsgemäss führt man die Umsetzung in dem Wirbelbett zunächst bei Temperaturen von ca.

20 bis 130, bevorzugt bei 70 bis 120, besonders bevorzugt bei 80 bis 100° C durch. Die Drucke betragen dabei 1 bis 50, bevorzugt 2 bis 10, besonders bevorzugt 3 bis 8 bar$_{abs}$.

Nachdem sich etwa 40, bevorzugt 50 bis 80%, der aromatischen Hydroxyverbindung mit dem Kohlendioxid zum entsprechenden Carbonat umgesetzt haben, wird die Temperatur in Abhängigkeit von dem jeweils eingesetzten Alkalisalz der aromatischen Hydroxyverbindung auf etwa 135 bis 300, bevorzugt 140 bis 210° C, erhöht und die Reaktion in Gegenwart von Kohlendioxid bei Drucken von etwa 1 bis 50, bevorzugt 3 bis 6 bar$_{abs}$, zu Ende geführt.

Diese Nachreaktion kann man auch in vorteilhafter Weise in einer anderen Apparatur, wie einem Rührwerksbehälter oder einer Schneckenmaschine, ggf. unter Zusatz geringer Mengen der entsprechenden aromatischen Hydroxyverbindung (etwa 5 bis 20 Mol-%, bezogen auf das eingesetzte Salz) separat durchführen.

In einer vorteilhaften Ausführungsform des erfindungsgemässen Verfahrens wird z.B. bei der Herstellung von Salicylsäure aus Natriumphenolat das bei der Nachreaktion erhaltene Reaktionsgemisch anschliessend noch bei höheren Temperaturen (etwa 180 bis 300° C), die wiederum von dem eingesetzten Alkalisalz der aromatischen Hydroxyverbindung abhängen, getempert.

Zur Aufarbeitung des Reaktionsgemisches wird ggf. zunächst unter Vakuum überschüssige aromatische Hydroxyverbindung abdestilliert. Das Reaktionsgemisch wird anschliessend mit Wasser versetzt, ggf. neutralisiert und durch Zusatzstoffe, wie Aktivkohle, geklärt und dann wird mit Mineralsäuren, wie Salzsäure oder Schwefelsäure, die freie Hydroxycarbonsäure gefällt.

Das erfindungsgemässe Verfahren kann wie folgt durchgeführt werden (gezeigt am Beispiel der Herstellung von Salicylsäure):

Das trockene, granulierte Natriumphenolat wird dem Wirbelbettreaktor kontinuierlich zugeführt und mit dem als Fluidisiergas dienenden Kohlendioxid, das im Kreis geführt wird, bei etwa 70 bis 90° C unter einem Druck von etwa 4 bis 6 bar$_{abs}$ umgesetzt. Unter Erhaltung dieses Gesamtdrucks wird das Kreisgas dabei jeweils um den Anteil an Kohlendioxid ergänzt, der bei der Umsetzung mit der Hydroxyverbindung verbraucht worden ist. Nachdem sich das Natriumphenolat zu ca. 70% durch Aufnahme von Kohlendioxid in das Natriumphenylcarbonat umgesetzt hat, wird das Carbonat in einen Rührwerksbehälter überführt und dort mit Kohlendioxid und unter Phenolzugabe bei Temperaturen von etwa 160 bis 170° C und bei einem Druck von etwa 5,5 bis 6,5 bar$_{abs}$ weiter zu dem Natriumsalicylat umgesetzt. In einer Nachreaktion bei etwa 190 bis 200° C und einem Druck von etwa 5,5 bar$_{abs}$ wird das bei der Reaktion in geringen Mengen entstandene Natriumsalz der p-Hydroxybenzoesäure in das Natriumsalz der Salicylsäure überführt. In einer sich anschliessenden Vakuumdestillation werden Restmengen an Phenol aus dem Reaktionsgemisch entfernt und anschliessend das Reaktionsgemisch in Wasser gelöst, neutralisiert, mit Aktivkohle geklärt und mit Schwefelsäure gefällt, wobei die freie Salicylsäure erhalten wird.

Das erfindungsgemässe Verfahren kann sowohl kontinuierlich als auch diskontinuierlich durchgeführt werden. Vorteilhafterweise wird es kontinuierlich durchgeführt.

Die nach dem erfindungsgemässen Verfahren erzielten Ausbeuten an aromatischen Hydroxycarbonsäuren betragen je nach eingesetztem Salz der aromatischen Hydroxyverbindungen etwa 85 bis 96% der Theorie, bezogen auf die Ausgangsverbindung.

Die Vorteile des erfindungsgemässen Verfahrens sind insbesondere zu sehen in der Vermeidung von lokalen Überhitzungen, wodurch eine Phenolbildung, die zu Verbackungen und Verklebungen führen würde, verhindert wird. Damit erreicht man wesentlich verkürzte Reaktionszeiten bei geringem Energieverbrauch. Durch die Möglichkeit einer genauen Temperaturführung kann die Bildung von Nebenprodukten minimiert werden. Ausserdem können mit dem erfindungsgemässen Verfahren Verfärbungen der Reaktionsprodukte durch Luftoxidation vermieden werden. Schliesslich werden nach dem erfindungsgemässen Verfahren die aromatischen Hydroxycarbonsäuren in guten Ausbeuten mit einer hohen Selektivität erhalten.

Bei dem erfindungsgemässen Verfahren überrascht besonders, dass das im Wirbelbett behandelte Granulat auch nach der Umsetzung mit dem Kohlendioxid in dieser Form erhalten bleibt und so ohne Probleme dem Wirbelbettreaktor entnommen werden kann. Ebenso bleibt die Granulatform bei der bei höherer Temperatur durchgeführten Vervollständigung der Reaktion erhalten, was besonders günstig für die technische Durchführung des erfindungsgemässen Verfahrens sowie für die Weiterverarbeitung der Reaktionsprodukte ist. Weiterhin ist es überraschend, dass trotz der Grobkörnigkeit der eingesetzten Alkalisalze aromatischer Hydroxyverbindungen keine Beeinträchtigung der Umsetzungsgeschwindigkeit und der Ausbeute eintritt, obwohl in „Indian J. Technol.", vol. 11, S. 187 (1973) darauf hingewiesen wird, dass mit zunehmender Teilchengrösse von Natriumphenolat die Reaktionsgeschwindigkeit der Carboxylierung abnimmt.

Die nach dem erfindungsgemässen Verfahren hergestellten aromatischen Hydroxycarbonsäuren sind wertvolle Zwischenprodukte für die Herstellung von Farbstoffen, Arzneimitteln, Pflanzenschutzmitteln, Gerbstoffen und Kosmetika („Ullmann's Encyclo. d. techn. Chemie", Bd. 13, 4. Aufl., S. 163-168).

Die nachfolgenden Beispiele sollen das erfindungsgemässe Verfahren verdeutlichen, ohne es jedoch auf diese Beispiele einzuschränken.

### Beispiel 1

Ein kontinuierlicher Strom von 8 Teilen/h trockenem Natriumphenolat in granulierter Form (Hauptanteil 0,5-1,5 mm Durchmesser) wird über eine geeignete Eintragungsvorrichtung in ein Wirbelbett eingetragen. Als Fluidisiergas dient Kohlendioxid, welches gleichzeitig Reaktionsgas ist. Das Reaktionsgas wird im Kreis gefahren und auf einen konstanten Druck von 5,5 $bar_{abs}$ gehalten. Das Gas nimmt die Reaktionswärme auf und wird nach Verlassen des Wirbelbettes in einem Wärmetauscher so weit abgekühlt, dass im Wirbelbett eine konstante Temperatur von 90° C aufrechterhalten bleibt. Pro Mol Natriumphenolat werden etwa 0,7 mol Kohlendioxid aufgenommen (Umsatz an aromatischer Hydroxyverbindung: 70%). Aus dem Wirbelbett werden mit einer Verweilzeit von ca. 20 min 10 Teile/h des Reaktionsproduktes im konstanten Strom entnommen. Dieses Produkt, das seine ursprüngliche Granulatform erhalten hat, fliesst kontinuierlich alternativ in einen von zwei Behältern mit einfacher Rühreinrichtung ein, welcher unter einem Kohlendioxiddruck von 5,4 $bar_{abs}$ steht. In diesem Behälter wird zunächst eine Temperatur von 160° C gehalten, bis der Behälter bis zu 85% mit Granulat gefüllt ist. Dann wird der Einlauf auf den zweiten Rührwerksbehälter umgeschaltet. Im ersten Behälter werden unter Rühren innerhalb 1 h 10-15 Mol-% Phenol (bezogen auf eingesetztes Natriumphenolat) eingedüst, das vom Granulat absorbiert wird. Unter einem Kohlendioxiddruck von 5,4 $bar_{abs}$ wird der Reaktorinhalt auf 210° C erhitzt und 1 h bei dieser Temperatur gehalten. Dann wird entspannt und überschüssiges Phenol unter Vakuum abdestilliert. Unterhalb 150° C wird der Kesselinhalt mit Wasser geflutet. Das gelöste Natriumsalicylat wird in bekannter Weise durch Ansäuern mit Mineralsäure in die freie Salicylsäure überführt. Die Ausbeute an Salicylsäure beträgt 94%, bezogen auf eingesetztes Alkali.

*Beispiel 2*

Ein kontinuierlicher Strom von 5 Teilen/h trokkenem, granuliertem Kalium-2-naphtholat wird in ein Wirbelbett eingetragen. Als Fluidisiergas dient Kohlendioxid, das im Kreis geführt und bei einem konstanten Druck von 5,5 bar gehalten wird. Das durch die Reaktion verbrauchte Kohlendioxid wird über die Druckhaltung ergänzt. Im Wirbelbett wird eine konstante Temperatur von 80° C gehalten, indem man das Kreisgas über einen Wärmetauscher abkühlt. Bei einer Verweilzeit von ca. 5 min werden etwa 0,5 mol Kohlendioxid pro Mol Naphtholat verbraucht (Umsatz: 50%). Aus dem Wirbelbett wird ein kontinuierlicher Strom von 5,6 Teilen/h Reaktionsprodukt entnommen, wobei die ursprüngliche Granulatform während der Reaktion erhalten bleibt.

Das Reaktionsprodukt wird in einem Rührwerksbehälter bei 80 bis 150° C unter einem Kohlendioxiddruck von 5,0 $bar_{abs}$ zu 2-hydroxynaphthalin-1-carbonsaurem Kalium umgesetzt. Die Ausbeute beträgt über 95%, bezogen auf eingesetztes Alkali.

Das Reaktionsprodukt besitzt eine hohe Reinheit und kann direkt als Zwischenprodukt weiter verarbeitet werden oder es wird in üblicher Weise in Wasser gelöst, neutral geklärt und mit Mineralsäuren in die freie Säure überführt.

**Patentansprüche**

1. Verfahren zur Herstellung von aromatischen Hydroxycarbonsäuren durch Umsetzung in fester Phase von Alkalisalzen aromatischer Hydroxyverbindungen mit Kohlendioxid in einem Wirbelbett, wobei man zunächst die Alkalisalze aromatischer Hydroxyverbindungen bei ggf. erhöhten Drücken bei Temperaturen von 20 bis 130° C umsetzt, bis sich mindestens 40% der Hydroxyverbindung in das entsprechende Carbonat umgewandelt haben, anschliessend die Reaktion bei Temperaturen von 135 bis 300° C zu Ende führt und danach das erhaltene Reaktionsprodukt in üblicher Weise in die freie Säure überführt, dadurch gekennzeichnet, dass man die Alkalisalze aromatischer Hydroxyverbindungen in Form von Granulat einsetzt.

2. Verfahren nach Anspruch 1, dadurch gekennzeichnet, dass man als Alkalisalze aromatischer Hydroxyverbindungen die Natrium- und/oder Kaliumsalze des Phenols, der Kresole, der Naphthole, des 2-Hydroxycarbazols oder des 3-Hydroxydiphenylenoxids einsetzt.

3. Verfahren nach den Ansprüchen 1 und 2, dadurch gekennzeichnet, dass man die Alkalisalze der aromatischen Hydroxyverbindungen in Form von Granulat mit einem Durchmesser von 0,2 bis 5 mm und einer inneren Oberfläche von 1 bis 6 m²/g einsetzt.

**Claims**

Process for the preparation of aromatic hydroxycarboxylic acids by reacting alkali metal salts of aromatic hydroxy compounds in the solid phase with carbon dioxide in a fluidised bed, wherein the alkali metal salts of aromatic hydroxy compounds are first reacted under, if appropriate, elevated pressures at temperatures of 20 to 130° C until at least 40% of the hydroxy compound have been converted into the corresponding carbonate, the reaction is then completed at temperatures of 135 to 300° C and then the reaction product obtained is converted, in a customary manner, into the free acid, characterised in that the alkali metal salts of aromatic hydroxy compounds are used in the form of granules.

2. Process according to Claim 1, characterised in that the sodium and/or potassium salts of phenol, cresols, naphthols, 2-hydroxycarbazole or 3-hydroxydiphenylene oxide are used as the alkali metal salts of aromatic hydroxy compounds.

3. Process according to Claims 1 and 2, characterised in that the alkali metal salts of the aromatic hydroxy compounds are used in the form of granules having a diameter of 0.2 to 5 mm and an internal surface area of 1 to 6 m²/g.

**Revendications**

1. Procédé de préparation d'acides hydroxycar- boxyliques aromatiques par réaction en phase solide de sels alcalins de composés hydroxylés aromatiques avec l'anhydride carbonique dans un lit tourbillonnaire dans lequel on fait d'abord réagir les sels alcalins des composés hydroxylés aromatiques éventuellement sous pression à des températures de 20 à 130° C, jusqu'à ce que 40% au moins du composé hydroxylé aient été convertis en le carbonate correspondant, puis on termine la réaction à des températures de 135 à 300° C, après quoi on convertit le produit de réaction obtenu de la manière habituelle en l'acide libre, ce procédé se caractérisant en ce que les sels alcalins de composés hydroxylés aromatiques sont mis en œuvre à l'état de granulés.

2. Procédé selon la revendication 1, caractérisé en ce que l'on met en œuvre, en tant que sels alcalins de composés hydroxylés aromatiques, les sels de sodium et/ou de potassium du phénol, des crésols, des naphtols, du 2-hydroxycarbazole ou de l'oxyde de 3-hydroxydiphénylène.

3. Procédé selon les revendications 1 et 2, caractérisé en ce que l'on met en œuvre les sels alcalins des composés hydroxylés aromatiques à l'état de granulés à un diamètre de 0,2 à 5 mm et à une surface interne de 1 à 6 m²/g.